# EUROPEAN PATENT SPECIFICATION

(11) **EP 3 628 250 B1**
(45) Date of publication and mention of the grant of the patent: **10.04.2024**
(21) Application number: 18197557.4
(22) Date of filing: 28.09.2018
(51) Int. Cl.: A61B 17/64

(54) **MEDICAL POSITIONING DEVICE**
MEDIZINISCHE POSITIONIERVORRICHTUNG
DISPOSITIF DE POSITIONNEMENT MEDICAL

(43) Date of publication of application: 01.04.2020
(73) Proprietor: PMU Innovations GmbH, 5020 Salzburg (AT)
(72) Inventor: Rodemund, Christian, 4060 Leonding (AT); Fierlbeck, Johann, 5020 Salzburg (AT)
(74) Representative: Vossius & Partner Patentanwälte Rechtsanwälte mbB

(56) References cited:
- GB-A- 538 599
- US-A- 2 346 346

## Description

The present invention relates to a medical positioning device that can be used in surgical procedures, particularly orthopedic procedures.

Several medical treatments require the positioning and/or repositioning of body parts of a patient. For example, if a patient suffers from a bone fracture, it may be necessary to reposition the fractured pieces of the bone in order to support and/or assure a healing process with the fractured pieces of the bone in the correct position.

A variety of fracture reduction devices are known in the art. For example, US 6,328,737 B 1 discloses a fracture reduction device comprising linear adjustment means for linearly reducing a fractured bone, and an angular adjustment mechanism for angularly reducing a fractured bone. However, the devices known in the art have different drawbacks. The devices are often large and heavy, and the handling is complicated and uncomfortable for a user, e.g. a physician.

US 2,346,346 A discloses an ambulatory fracture reduction and immobilization splint comprising complemental yokes for embracing a limb, at opposite sides of a fracture, transfixions mounted by and extended between the legs of each yoke for transfixing the corresponding bone fragment for reduction of the fracture, by immobilization of the yokes, each yoke having ball sockets formed therein, a split ball bearing rotatably confined in each socket, connector rods extended along the limb between the yokes and slidably passing through corresponding ball bearings therein for adjustment of the yokes therealong for their change in spacing and angular relationship as required for and during reduction of the fracture, and means adapted to be tightened against the said balls to fix them against rotation, and to clamp them on the rods to fix the yokes in their adjustment relative to each other.

GB 538 599 A discloses a fracture apparatus comprising a pair of yoke members and skeletal pins clamped by said yoke members and adapted to be passed completely through a bone, said yoke members being connected by adjustable rods to maintain them in a predetermined relation characterized in that each of said yoke members has at one end a pair of clamping plates co-operating with one another to clamp one end of each of a pair of skeletal pins, and at the other end a seat adapted to secure the other end of one of said pins and also a pivotally connected clamping member to secure the other end of the second skeletal pin.

It is therefore an object of the present invention to provide a medical positioning device with improved functionality. This is achieved with the inventive medical positioning device according to the claims.

In the following description, the invention will be described with reference to a treatment of a fractured foot. However, the invention is applicable to other repositioning treatments, e.g., of other bones, without departing from the scope of the invention.

The invention is defined by independent claim 1. Optional features are defined by the dependent claims.

A medical positioning device according to the invention comprises a first carriage assembly configured for mounting a first pin, wherein the spatial orientation of the first pin is adjustable by means of a first spherical joint with three rotational degrees of freedom with an angular range of at least ± 10° each. The device further comprises a second carriage assembly configured for mounting a second pin, wherein the spatial orientation of the second pin is adjustable by means of a second spherical joint with at least three rotational degrees of freedom with an angular range of at least ±10° each. Furthermore, the device has an extension assembly configured for adjusting a distance and optionally a spatial orientation between the first pin and the second pin, wherein only one of the carriage assemblies comprises an extension assembly.

Particularly, the medical positioning device may be configured for repositioning bones in a patient's body part, preferably for repositioning bones in a human's foot. In the context of the present invention the term "bones" is not only used for complete bones but also for portions or pieces thereof.

The pins serve for attaching the medical positioning device to a patient's body part, e.g., one or more bones, that needs to be repositioned. The pins may be suitable for and configured for being inserted into the patient's body and may be particularly suitable for being inserted into and through a patient's bone. Methods for inserting a pin into a patient's body are known in the art and will therefore not be described in detail. Particularly, the first pin is configured for being inserted on a first side of the body part and the second pin is configured for being positioned at a second side of the body part opposite the first side, such that the body part to be treated is located between the two pins. With this configuration, the medical positioning device may be used to apply forces and moments to the body part between the pins, which enables a user to rearrange the body part, e.g., fractured bone pieces, between the pins by actuating an extension mechanism of the medical positioning device.

In order to perform the rearrangement of the body part between the pins, the first and second pins need to be moved relative to each other. The medical positioning device comprises first and second carriage assemblies for mounting the first and second pins, respectively. The first and second carriage assemblies comprise first and second spherical joints, respectively. The spherical joints may be configured for enabling spatial reorientation of the pins. The spherical joints, and thus the pins, each have three degrees of freedom, as commonly known for spherical joints. Typically, the range of movement of a spherical joint is limited by the specific geometry of the spherical joint, for example, the configuration of the ball's attachment structure for connection to another member and of the socket of the spherical joint. In the context of the present invention, a spherical joint preferably has a rotational range of 360°, i.e., no limitations, for rotation about its longitudinal axis created by the ball and socket of the joint. The other two rotational or pivotable degrees of freedom, which are preferably rotations or pivotal about two axes that are perpendicular to each other and perpendicular to the longitudinal axis of the spherical joint, have an angular range of at least ± 10° each, preferably an angular range of at least ± 20° each, more preferably an angular range of at least ± 30° each. This enables an angular adjustment of the pins and thus the bones. The maximum angular range for the other two rotational or pivotable degrees of freedom may be ± 70° each, or ± 60° each, or ± 50° each. Also, ranges from ± 10°, ± 20°, or ± 30° up to ± 70°, or up to ± 60°, or up to ± 50° (all permutations) are envisaged.

The medical positioning device further comprises an extension assembly. By actuating the extension assembly, a user may adjust the distance between the first carriage assembly and the second carriage assembly, thereby adjusting the distance between the first pin and the second pin. This may be achieved by a linear movement of the first carriage assembly relative to the second carriage assembly.

Hence, the medical positioning device may enable an adjustment of the orientation of each of the pins as well as their distance to each other, which allows for a precise repositioning of the attached body parts

The extension assembly may be configured for adjusting the distance of the carriage assemblies of the first and second pins relative to each other in a range of at least 2 cm, or at least 4 cm and/or up to 10 cm, or up to 8 cm, or up to 5 cm. Again, all combinations (permutations) of the various lower and upper limits are envisaged.

The medical positioning device, and particularly the spherical joint(s), may be configured for resisting a moment of at least 3 Nm, preferably at least 9.5 Nm, more preferably at least 11 Nm, most preferably at least 12.5 Nm, as applied by the pins. This ensures that the pieces of a fractured bone may be repositioned into the correct locations, even against the forces exerted on the pieces, e.g., by surrounding tissue and during the distraction process.

At least one of the first and second carriage assemblies may comprise a support member configured for mounting the respective pin, for example, at two mounting sites. The mounting sites are preferably separated by 7 to 20 cm, more preferably by 10 to 14 cm. Preferably, both carriage assemblies comprise a respective support member.

The pins may be configured for traversing a patient's body part. The mounting sites of the support member(s) may be configured to be located on different sides of the traversed body part. For example, one of the pins may be configured for traversing the patient's foot from a lateral to a medial side such that one mounting site is located laterally of the foot and the other mounting site is located medially of the foot.

The medical positioning device may comprise a base frame. The first carriage assembly and the second carriage assembly may each be adjustably mounted to the base frame, preferably via first and second carriages, respectively. The base frame may comprise two rods that serve as rails for the first and second carriages. The carriage assemblies, preferably the carriages, may each comprise a fixing assembly for removably fixing the carriage assembly to the base frame. The fixing assembly may comprise a screw, a quick release skewer or the like. The removable fixing assembly ensures that the distance between the carriage assemblies may be adjusted, e.g., for adjusting the medical positioning device to the size of the body region to be treated. Alternatively, one of the carriage assemblies may be non-adjustably mounted to the base frame, preferably via the respective carriage.

Preferably, each support member is mounted to the respective carriage via the respective spherical joint. The at least one support member, preferably both support members, may have any suitable form. Preferably, the form is generally C-shaped, U-shaped, V-shaped or T-shaped. The at least one support member preferably comprises one or more brackets configured for mounting the respective pin at the respective mounting site. Preferably, the at least one support member, preferably each support member, comprises two brackets, one for each mounting site for the associated pin. The bracket's position on the support member may be adjustable. This may provide further flexibility to the device.

Preferably, the support member comprises a spring-screw-mechanism, wherein the spring attaches the bracket to the rest of the support member but allows for a movement of the bracket relative to the rest of the support member that enables the user to insert the pin into an engagement region for the pin. The screw is preferably configured for closing the bracket, thus causing the bracket to fix the support member at the pin

The fixation of the pin may be configured for reducing bending of the pin. The support member and the bracket may together form a three point fixation of the pin at each mounting site. This leads to a decrease in bending of the pin in comparison to a single point fixation, for example a hole, at each mounting site. This may be advantageous because the pretension adds stability to the pin. Thus, the pin is less prone to an unwanted deformation due to the forces and moments acting on the pin during the repositioning procedure. It is preferable to have the pin as straight as possible because this helps the physician to identify the position and orientation of the pin inside the patient's body and thus the orientation and position of the body parts to be repositioned. Also a straight pin reduces the deformation of bone volume where the pin penetrates the respective bone. This increases a stable fit of the pin in the bone.

The base frame of the medical positioning device may generate an area and/or volume that is at least partially surrounded by the base frame. For example, if the base frame comprises the two rods as mentioned above, the two rods define an area between them. Similarly, if the base frame comprises three rods, the three rods define a volume between them that has the shape of a prism with a triangular base. The area and/or the volume generated by the base frame is/are preferably free of the pins. This has several advantages. For example, it increases the number of possible orientations that the medical positioning device may have relative to the body portion to be treated. This makes it easier for the user to appropriately attach the device to the patient's body. Furthermore, it increases the adjustability to different body parts such that the device may be not only used for one specific type of repositioning treatment, e.g., repositioning of the fractured bone pieces of a foot, but for several different repositioning treatments of different body types, e.g., also for bones of a leg and/or bones of an arm and/or bones of a hand.

The extension assembly is preferably associated with the first carriage assembly and forms a first extension assembly. Preferably, it comprises a threaded rod coupled to the support member of the first carriage assembly, wherein the medical positioning device is preferably configured for adjusting the distance between the first and second pins by rotational movement between the threaded rod and a counter threading. For example, the first carriage assembly, preferably the carriage of the first carriage assembly, may comprise a screw fence, and the threaded rod is attached to the spherical joint.

According to the present invention, only one of the carriage assemblies comprises an extension assembly.

Additionally or alternatively, the second carriage assembly may comprise a force sensor or dynamometer. The force sensor or dynamometer may serve to measure and display a force exerted between the first and second carriage assemblies during a repositioning procedure. The force sensor or dynamometer may comprise a spring and use the principle of a spring balance. However, other force sensors such as a force sensing resistor, an eddy current type absorber, a powdered dynamometer, hysteresis dynamometer, an electric motor/generator dynamometer, and the like are contemplated.

Preferably, each of the spherical joints is arranged to couple the respective support member to the respective carriage, thus providing the flexibility to the system. Preferably, at least one spherical joint, more preferably each of the spherical joints, comprises a ball, a corresponding socket and a locking mechanism configured for reversibly receiving and locking the ball in the socket. This provides additional flexibility to the system. For example, the ball may be attached to the support member, whereas the socket may be coupled to the carriage. With this embodiment, the user may first attach the pins to the body of the patient and then attach the first and second support members to the first and second pins, respectively. Subsequently, the user may attach the first and second support members to the first and second carriages, respectively. This may be preferably achieved by inserting the ball of the spherical joint (located on the support member) into the corresponding socket of the spherical joint (located on the carriage) and locking the ball in the socket by means of the locking mechanism. Ball and socket may also be arranged vice versa, i.e., the ball may be attached to the carriage and the socket may be attached to the support member.

If the socket is configured for being attached to the support member, the threaded rod may be coupled to the ball, i.e., the shaft of the ball, and if the ball of the spherical joint is configured for being attached to the support member, the threaded rod may be coupled to the socket.

The locking mechanism may be implemented in any suitable manner. Preferably, the socket comprises a side opening for inserting the ball such that insertion and removal of the ball into and from the socket is only possible through the side opening of the socket but not in the longitudinal direction. The spherical joint preferably further comprises a locking ring that may be displaced between a closing position and opening position. The spherical joint may comprise a sleeve along a surface of the socket, preferably along the outer surface of the socket and even more preferably along the inner surface of the socket. The sleeve may be configured for at least partially closing the side opening of the socket, when the locking ring is in a closing position, such that the ball may not exit through the side opening. If the sleeve/locking ring is, however, in the opening position, the side opening is not blocked by the sleeve and the ball is free to leave the socket. The locking ring may be biased to the closing position, e.g., by means of a spring. The locking ring may preferably be fixed to the socket with the locking ring - socket - assembly being configured to move relative to the sleeve. Alternatively, the locking ring may be fixed to the sleeve with the locking ring - sleeve - assembly being configured to move relative to the socket.

In an exemplary method of applying the device in a repositioning treatment, which does not form part of the invention, a user may first attach the first and second pins at appropriate positions in the patient's body. Then, the user may attach first and second support members to first and second pins, respectively. The user may couple the first and second support members to the first and second carriages, respectively. The coupling of the support members to the respective carriage may occur by coupling of the respective ball and the corresponding socket of the respective spherical joint. As already mentioned, the ball part of the spherical joint may be attached to the support member and the socket part of the spherical joint may be attached to the carriage, or vice versa. First and second carriages may then be coupled to the base frame.

In a preferred embodiment, the base frame or the carriages may be, directly or indirectly, coupled to a fix member that is fixedly installed, such as an operation table, a wall, a floor, a ceiling and the like. In the case of an indirect coupling to the fix member, any suitable intermediate fixation device may be used.

Further details of the invention will be described with reference to the figures, in which:
- Fig. 1A: shows a perspective view of an embodiment of the medical positioning device;
- Fig. 1B: shows the medical positioning device of Figure 1A in the same view, but attached to a human's foot;
- Fig. 2A: shows the embodiment of Figures 1A and 1B in a view at the first carriage along a longitudinal direction from the first to the second carriage of the device;
- Fig. 2B: shows the embodiment of Figures 1A-2A in a view at the second carriage along the longitudinal direction of the device in an opposite direction as compared to Fig. 2A;
- Fig. 2C: shows the embodiment of Fig. 1A-2B in a view along a vertical direction perpendicular to the longitudinal direction of the device;
- Fig. 2D: shows the embodiment of Fig. 1A-2C in a view along the width direction perpendicular to the longitudinal direction of the device and the vertical direction;
- Fig. 2E: shows the embodiment of Fig. 1A-2D in a view along the width direction of the device in an opposite direction as compared to Fig. 2D;
- Fig. 3: shows a cross section through a spherical joint.

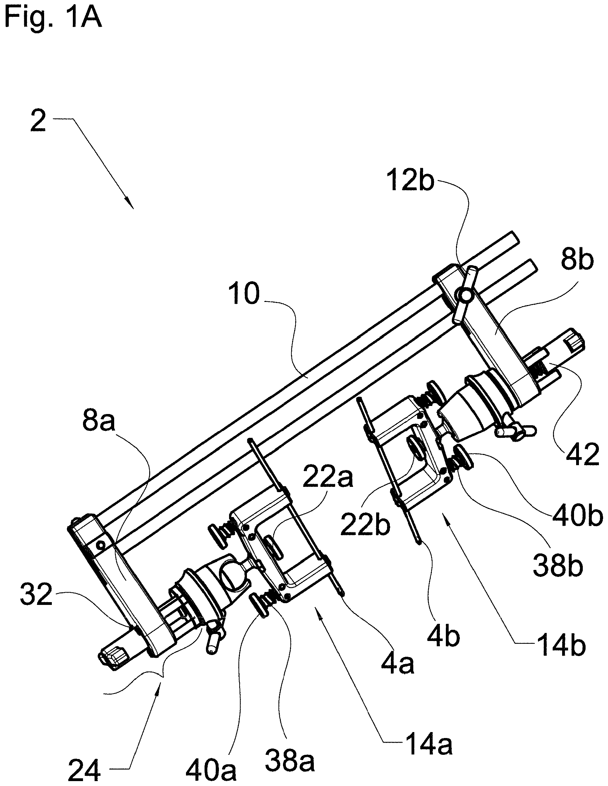

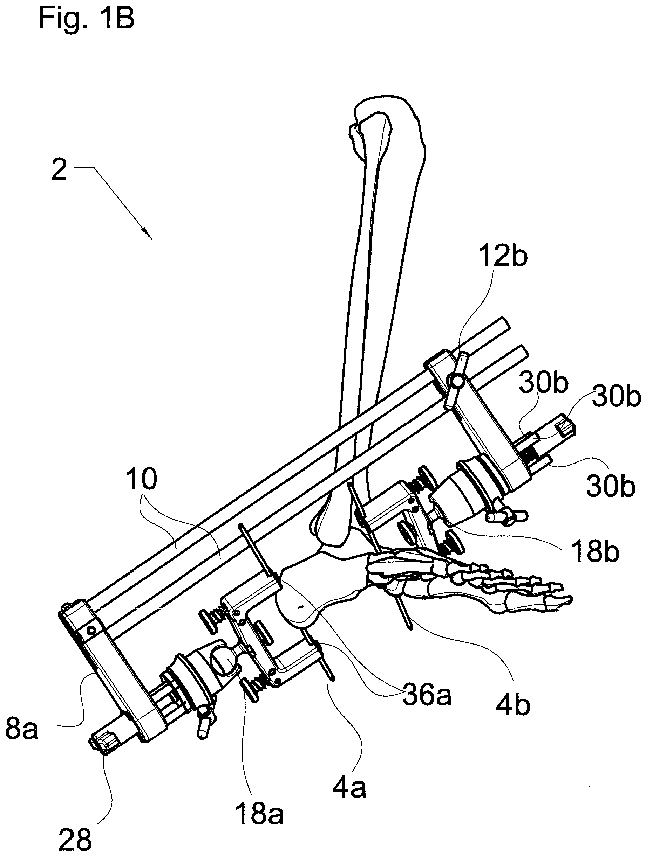

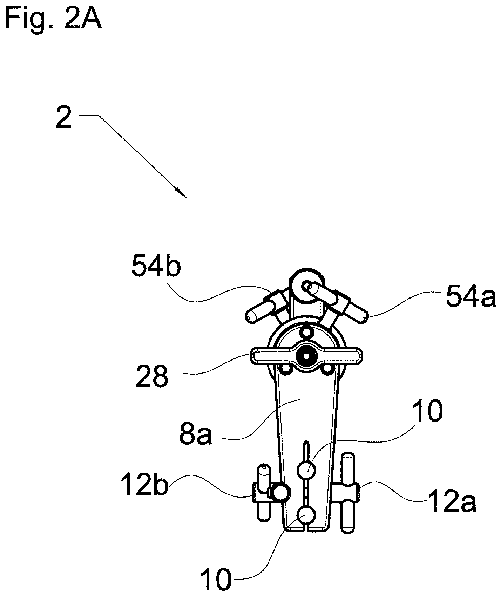

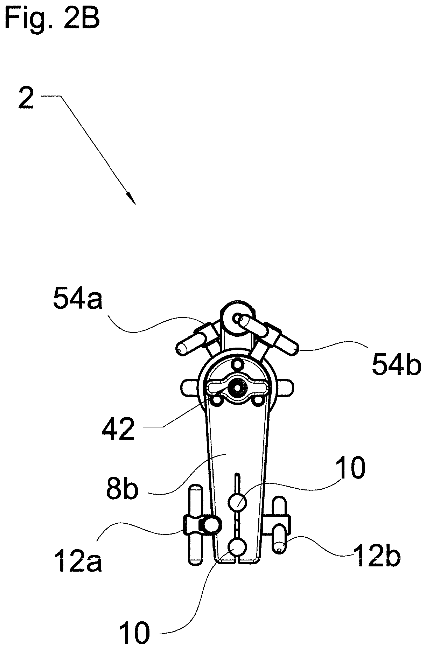

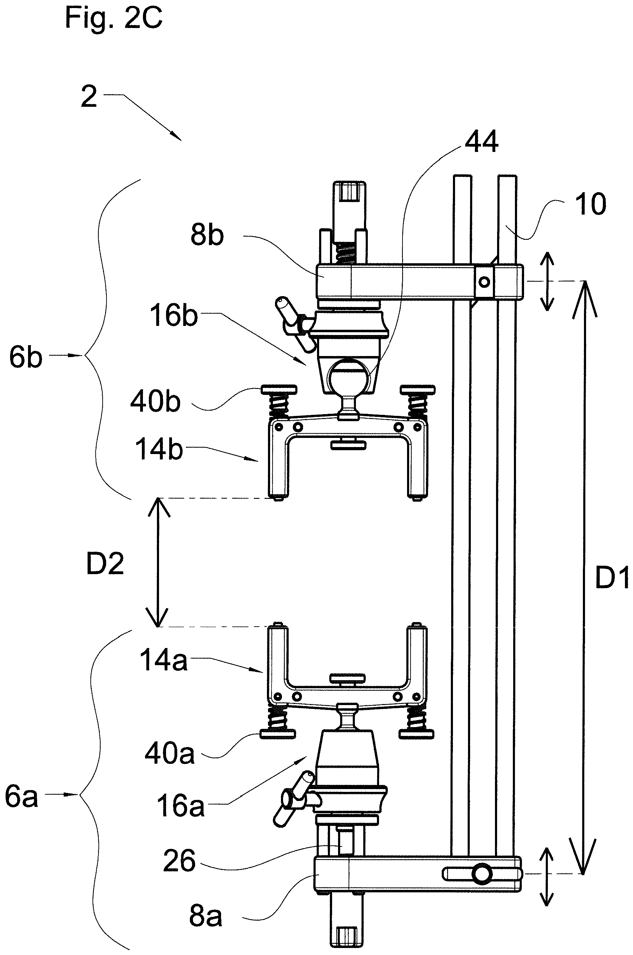

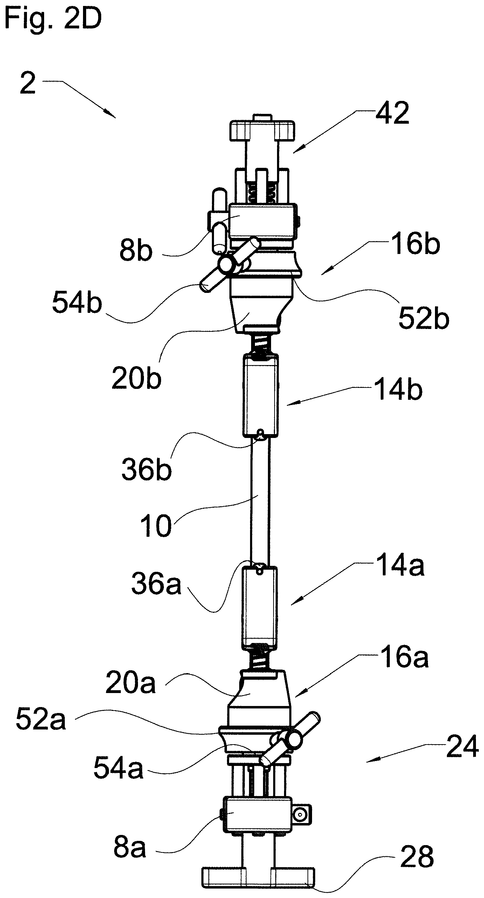

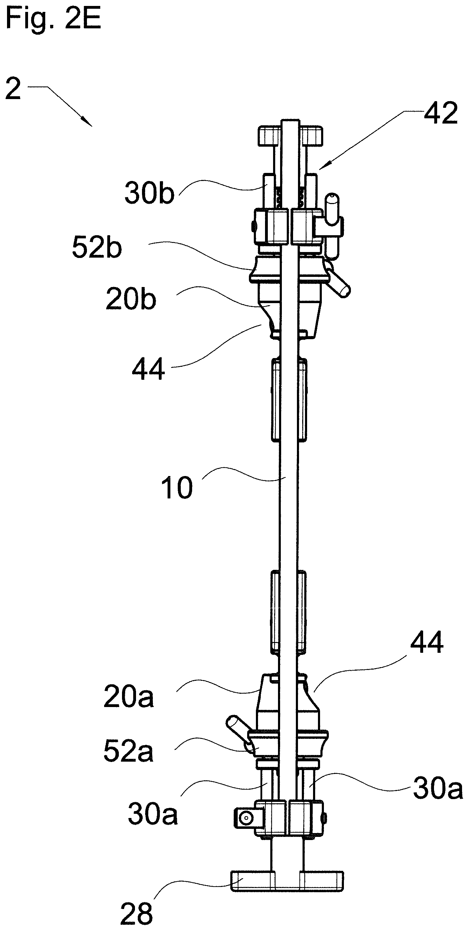

Figures 1A - 2E show different views of a preferred embodiment of a medical positioning device 2 according to the present invention. Figures 1A and 1B show the same perspective view of the device 2, once with the device installed at a human's foot (only the bones of the shank and the foot are shown) and once the device without the human's foot. With respect to the foot in Figure 1B, the view is an angled bottom up view. Figures 2A - 2E show the embodiment of Figures 1A - 1B in several views along different axes through the device for further clarification. The medical positioning device 2 is shown with first and second pins 4a, 4b coupled to first and second carriage assemblies 6a, 6b, respectively. Each carriage assembly 6a, 6b may comprise a carriage 8a, 8b configured for coupling to a base frame 10 (see Fig. 2C). The base frame 10 of the shown embodiment comprises two rods, however, other constructions, e.g., a base frame comprising one rod, three rods, four or more rods and the like, are also contemplated.

Each carriage 8a, 8b may comprise an appropriate number of seats for receiving the respective counterpart of the base frame 10. The illustrated embodiment has two rods of base frame 10 such that movement of the carriage 8a, 8b relative to the base frame 10 is enabled. The carriages 8a, 8b may slide along base frame 10 as indicated by the double arrows in Figure 2C. This allows for a coarse adjustment of the distance D1 between the first and second carriages 8a, 8b and thus the whole carriage assemblies 6a, 6b. Thus, a user may attach the medical positioning device 2 via the pins 4a, 4b to different body parts, e.g. a foot, a hand, an arm or a leg, and/or two different patients, e.g. an adult or a child, a tall person or a small person.

Once the carriages 8a, 8b are appropriately positioned on the base frame 10 and with respect to one another, the carriages 8a, 8b may be locked by means of a fixing assembly 12a, 12b, such that they cannot move relative to the base frame 10 anymore. As a fixing assembly, any appropriate mechanism may be used. However, a fixing assembly 12a, 12b including a screw for tightening the carriages 8a, 8b around the rods of the base frame 10 is preferred. For example, a quick release skewer may be used.

At least one of the carriage assemblies 6a, 6b may comprise a support member 14a, 14b. Preferably, as shown in the Figures, both carriage assemblies 6 comprise a respective support member 14a, 14b. The support members 14a, 14b may be configured for coupling a respective pin 4a, 4b, preferably at two mounting sites.

At least one of the support members 14a, 14b, preferably (and as shown in the Figures) both support members 14a, 14b are coupled to the corresponding carriage 8a, 8b via a respective spherical joint 16a, 16b (see Fig. 2C). In the embodiment shown in the Figures, the balls 18a, 18b of the spherical joints are coupled to the support members 14a, 14b, while the sockets 20a, 20b of the spherical joints 16a, 16b are coupled to the carriages 8a, 8b. However, this arrangement may be vice versa for one/all spherical joint/s. Coupling of the ball 18a, 18b to the support member 14a, 14b may be accomplished by any suitable means. In the embodiment of the Figures, the balls 18a, 18b are coupled to the corresponding support members 14a, 14b by means of a respective screw 22, particularly a nut 22.

The coupling of the support member 14a, 14b to the carriage 8a, 8b via the spherical joint 16a, 16b allows for an adjustment of the orientation of the support member 14a, 14b relative to the respective counterpart of the spherical joint 16a, 16b (socket 20a, 20b) and thus the respective carriage 8a, 8b, and thus via the base frame 10 also relative to the other carriage assembly 6a, 6b with the other support member 14a, 14b.

The first carriage assembly 6a (see Fig. 2C; shown to the left in Figures 1A, 1B and to the bottom in Figs. 2C-2E) comprises an extension assembly 24 configured for adjusting a distance D2 between the first and second support members 14a, 14b and thus the first and second pins 4a, 4b. The extension assembly 24 may comprise any suitable mechanism. For example, the extension assembly 24 may include a threaded rod 26 and an extension screw 28 mounted on the threaded rod. The threaded rod 26 may be coupled to the socket 20 of the spherical joint 16a. The socket 20a of the spherical joint 16a may be further coupled to at least one, preferably at least two, more preferably at least three guide rods 30. The carriage 8a may comprise a corresponding number of seats, particularly holes, particularly through holes, for receiving the guide rods 30.

The extension assembly 24 may be configured such that actuating the extension screw 28 results in a longitudinal movement of the socket 20a and thus the support member 14a and the pin 4a relative to the base frame 10. The direction of the movement may depend on the rotating direction of the screw 28. The longitudinal movement of the socket 20a may be achieved, e.g., by having the extension screw 28 and the socket 20a on opposing sides of the carriage 8a with the threaded rod 26 traversing the carriage 8a. In this configuration, the carriage 8a acts as a screw fence 32 for the extension screw 28. The threaded hole that forms the counterpart to the threaded rod 26 and translates the rotational movement of the extension screw 28 into a longitudinal movement of the socket 20a may be located in any suitable member. For example, it may be located in the extension screw 28 or in the carriage 8a.

The extension assembly 24 may comprise a locking mechanism (not shown) that may be configured for preventing a longitudinal movement of the support member 14a. In the embodiment shown in the figures, the locking mechanism may prevent a rotation of the extension screw 28. This can be achieved by the interaction of the pitch of the threaded rod 26 and the extension screw 28 to generate a self-blocking thread. Alternatively any mechanism to increase friction within the system of the extension assembly 24 may be suitable to achieve a locking mechanism.

The support members 14a, 14b may be configured for mounting the respective pin 4a, 4b. Preferably, the support members 14a, 14b are configured for mounting the respective pin 4a, 4b at two mounting sites, although other numbers of mounting sites are also contemplated. The attachment of the pin 4a, 4b at the mounting sites may be implemented in any suitable way. For example, the support member 14a, 14b may comprise a bracket 36 at each mounting site, i.e. two brackets 36 for each support member 14a, 14b in the embodiment shown in the Figures. Each bracket 36 may be configured for clamping the respective pin 4a, 4b at the respective mounting site. Preferably, each bracket 36 is coupled to a bracket spring 38 and a bracket screw 40. The bracket spring 38 may be configured for securing the bracket 36 to the support member, while still allowing the bracket 36 to be opened far enough to insert the pin 4a, 4b. Preferably, the bracket spring 38 biases the bracket 36 to a closed position and the bracket 36 may be brought into an open position for inserting the pin 4a, 4b by actuating the spring, e.g., by pushing the bracket screw 40 towards the support member 14a, 14b.

With this configuration, the support member 14a, 14b may be loosely mounted to the pin 4a, 4b in a way that still allows for a movement of the support member 14a, 14b in the mounting site but prevents the support member 14a, 14b from unintentionally falling off the pin 4a, 4b. After this initial loose attachment, the support member 14a, 14b may be fixed in its final position by actuating the bracket screw 40. The bracket screw 40 may be configured for tightening the bracket 36, which may result in the support member 14a, 14b being fixedly clamped to the pin 4a, 4b through the bracket 36. In this way, the risk of dropping the support member 14a, 14b during the procedure of attachment to the pin 4a, 4b is significantly reduced. This is particularly relevant for medical applications, such as the medical applications of the positioning device of the present invention, in which dropping of a device such as the support member 14a, 14b may result in severe consequences for the patient. Moreover, the above mounting mechanism provides for a flexible and comfortable mounting of the support member 14a, 14b to the pin 4a, 4b, as the support member 14a, 14b may be first attached at the first mounting site and then attached at a second mounting site. Throughout the attachment procedure, the pin 4a, 4b may be repositioned relative to the support member 14a, 14b, thus providing for optimal positioning of the pin 4a, 4b relative to the support member 14a, 14b.

Preferably, the support member may apply a pretension to the pin 4a, 4b, when the pin 4a, 4b is fixed via the brackets 36 by the described three point fixation. Applying pretension to the pin 4a, 4b makes the pin 4a, 4b more stable during the procedure of repositioning.

Preferably, the bracket's position on the support member may be adjustable. For example, the support member may comprise a movable mount (not explicitly shown in the Figures) that comprises the bracket. The mount may be configured to be fixed in different positions relative to the support member 14a, 14b.

According to the invention, the second carriage assembly 6a does not comprise the extension assembly 24. Instead, the support member 14b may be coupled to the carriage 8b, either directly (resulting in a fixed coupling) or via spherical joint 16b, which enables a rotational movement but no longitudinal movement.

Additionally or alternatively, one of the carriage assemblies 6a, 6b may comprise a force sensor 42 (dynamometer), configured for measuring an applied force. The force sensor 42 may be of any suitable type, e.g., one of the force sensors already mentioned. For example, the force sensor 42 may use the principle of a spring balance. For example, the force sensor 42 may comprise a spring and a corresponding scale on, e.g., one or more of the corresponding guiding rods 30. If a longitudinal force is applied on the corresponding pin 4a, 4b, the spring is extended (or contracted) proportional to the applied force in accordance with Hook's Law and this expansion is displayed to the user by the corresponding scale, which translates the expansion of the spring into the corresponding amount of force.

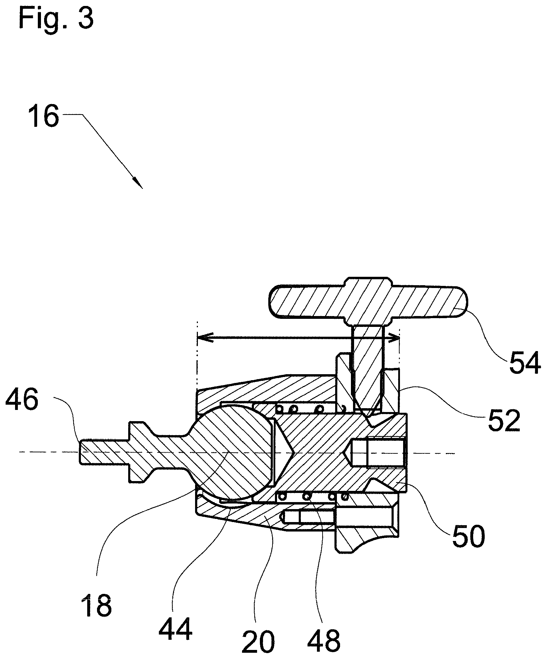

Any suitable spherical joint may be used in the invention. However, Figure 3 shows a cut through a preferred embodiment of the spherical joint 16 including ball 18 and socket 20 according to the invention. The socket 20 is configured for receiving the ball 18. The ball 18 may be inserted into the socket 20 through a side opening 44. The ball 18 may comprise a shaft 46 that extends from the ball 18 and that is configured for being attached to the appropriate member, preferably the support member 14. Note that the ball 18 with the shaft 46 may also be configured for attachment to the carriage 8 and/or the extension rod 26. The spherical joint 16 may comprise a joint locking mechanism. The joint locking mechanism serves for locking the ball 18 in the socket 20 when in the closing position and for allowing the ball 18 to leave the socket 20 when in an opening position.

The exemplary and inventive joint locking mechanisms shown in the Figures, and particularly in Figure 3, comprises a joint spring 48, a sleeve 50, a locking ring 52 and a joint screw 54. In a preferred embodiment as shown in figure 3, the locking ring 52 is coupled to the socket 20. This coupling may be implemented by any suitable means, for example, one or more threading, screws, nails, rivets, clamps and/or welds and/or the like. The locking ring - socket - assembly is arranged surrounding the sleeve 50 and movable relative to the sleeve 50 as indicated by the double arrow in Figure 3. If the locking ring - socket - assembly is in the closing position, the sleeve 50 blocks the opening 44 of the socket 20, thus preventing the ball 18 from leaving the socket 20 through opening 44. If the locking ring - socket - assembly is, however, in the opening position, the sleeve 50 does not block the opening 44, thus allowing the ball 18 to leave the socket 20 through opening 44. The locking spring 48 may be configured for biasing the locking assembly into the closing position. Thus, a force needs to be applied in order to switch the locking ring - socket - assembly into the opening position to enable the ball 18 to escape the socket 20. This advantageously provides for a security mechanism.

The joint locking mechanism may comprise a joint screw 54 for locking the locking ring 52 with the sleeve 50, thus inhibiting a movement of the locking ring - socket - assembly into the opening position.

According to a preferred embodiment and as shown in the Figures, the medical positioning device is constructed such that the pins do not interfere with the base frame. Preferably, the base frame defines an area and/or a volume that is at least partially surrounded by the base frame and that is free of the pins. In the case of the preferred embodiment shown in the Figures, the two rods of the base frame 10 define an area A between them. This area A is free of the pins 4a, 4b when the medical positioning device is coupled to the pins 4a, 4b. This provides the advantage that the medical positioning device may be comfortably arranged around the body portion to be treated without the problem of taking care that the base frame actually fits around the body portion. Furthermore, the medical positioning device may be attached to and removed from the pins and thus the body without the necessity of disassembling the medical positioning device.

Preferably, the pins 4a, 4b are configured for traversing a patient's body part. Preferably, the mounting sites are configured to be located on different sides of the traversed body part. For example, the pins 4a, 4b may be configured for traversing a human's foot. For example, the first pin 4a may be configured for traversing the Calcaneus and the second pin 4b may be configured for traversing the Talus, e.g., as shown in Figure 1B, or vice versa. In the case shown in Figure 1B, first mounting sites of the pins 4a, 4b are located on a lateral side of the foot and second mounting points of the pins 4a, 4b are located on a medial side of the foot.

The preferred embodiment as described above may be used according to the following method, which does not form part of the invention.

In general, a preferred embodiment of the invention is very flexible in terms of the attachment procedure, i.e. the procedure of attaching the medical positioning device to a patient. This enables a user to use the medical positioning device for many different specific applications such as different body parts of different types of patients. A user, e.g. a physician, may start with assembling the complete medical positioning device from its individual members and then attach the completely assembled medical positioning device to pins in the patient's body portion to be repositioned. Alternatively, the user may assemble the medical positioning device on site, i.e. the user only pre-assembles none, one or more parts of the medical positioning device and assembles these (pre-assembled) parts, e.g. one after the other, to the pins and/or those parts of the device that have already been assembled to the pins.

Referring again to the preferred embodiment shown in the Figures, a user may preferably attach first and second pins 4a, 4b to a patient's body as required from a medical point of view. The user may then assemble the first and second support members 14a, 14b to the first and second pins 4a, 4b, respectively. The support members 14a, 14b may already have the balls 16a, 16b coupled to them or the user may now couple the balls 16a, 16b to the support members 14a, 14b. The user may couple the first socket 20a to the first ball 16a of the first support member 14a and the second socket 20b to the second ball 16b of the second support member 14b. The user may then attach the first carriage 8a to the first spherical joint 16a, i.e., the first socket 20a, and the second carriage 8b to the second spherical joint 16b, i.e., the second socket 20b. Alternatively, the user may pre-assemble the carriage 8a, 8b with the socket 20a, 20b and assemble the carriage - socket - assembly as one piece to the ball 16a, 16b. In any case, the user may then couple the first and second carriages 8a, 8b with the base frame 10. This procedure will automatically result in the first and second carriages 8a, 8b having the correct distance D1. Alternatively, the user may pre-assemble the base frame 10 with one or both of the carriages 8a, 8b and optionally one or both of the sockets 20a, 20b.

With the device 2 attached to the pins 4a, 4b through the patient's body, the user may then actuate the extension member 24, e.g., the extension screw 28, thus causing the carriage assemblies 6a, 6b (and thus the pins 4a, 4b) to move away from each other. Thereby, the body parts of the patients are repositioned according to the actuation of the extension screw 28.

In a further alternative, the user may even pre-assemble the base frame 10 with one or both of the carriages 8a, 8b, optionally one or both of the sockets 20a, 20b, optionally one or both of the balls 16a, 16b, and optionally one or both of the support members 14a, 14b. In this case, the user may set the distance D1 to an appropriate value upon coupling the medical positioning device to the pins 4a, 4b.

In a preferred embodiment, the base frame 10 may be, directly or indirectly, coupled to a fix member that is fixedly installed, such as an operation table, a wall, a floor, a ceiling and the like. In the case of an indirect coupling to the fix member, any suitable intermediate fixation device may be used, e.g., a flexible mounting arm for surgical devices. This provides additional stability to the system.

A person skilled in the art will recognize that the specific sequence of assembling the individual components of the medical positioning device may vary depending on the specific application of the device.

## Claims

1. A medical positioning device (2), comprising:
a first carriage assembly (6a) configured for mounting a first pin (4a), wherein the orientation of the first pin (4a) is adjustable by means of a first spherical joint (16a) with three rotational degrees of freedom with an angular range of at least ±10° each;
a second carriage assembly (6b) configured for mounting a second pin (4b), wherein the orientation of the second pin (4b) is adjustable by means of a second spherical joint (16b) with three rotational degrees of freedom with an angular range of at least ±10° each;
an extension assembly (24) configured for adjusting a distance (D2) between the first pin (4a) and the second pin (4b);
wherein only one of the carriage assemblies (6) comprises an extension assembly (24).

2. The medical positioning device (2) of claim 1, wherein the extension assembly (24) is configured for adjusting a distance (D2) between the first carriage assembly (6a) and the second carriage assembly (6b), thereby adjusting the distance between the first pin (4a) and the second pin (4b).

3. The medical positioning device (2) of claim 1 or 2, wherein the extension assembly (24) is configured for adjusting the distance between the first and second pins relative to each other in a range of at least 2 cm, or at least 4 cm, and/or up to 10 cm, or up to 8 cm, or up to 5 cm.

4. The medical positioning device (2) of any of the preceding claims, wherein the first carriage assembly (6a) and/or the second carriage assembly (6b) are configured for resisting a moment of at least 3 Nm, preferably at least 9.5 Nm, more preferably at least 11 Nm, most preferably at least 12.5 Nm, applied by the pins (4a, 4b), respectively.

5. The medical positioning device (2) of any of the preceding claims, further comprising a base frame (10), wherein the first carriage assembly (6a) and the second carriage assembly (6b) are adjustably mounted to the base frame (10) via first and second carriages (8), respectively; or wherein one of the first and second carriage assemblies (6) is adjustably mounted to the base frame (10) via the respective carriage (8) and the other carriage assembly (6) is non-adjustably mounted to the base frame (10) via the respective carriage (8).

6. The medical positioning device (2) of any of the preceding claims, wherein at least one of the first and second carriage assemblies (6) comprises a support member (14) configured for mounting the respective pin (4) at two mounting sites, wherein the mounting sites are preferably separated by 7 to 20 cm, more preferably 10 to 14 cm.

7. The medical positioning device (2) of claim 6, wherein the pins (4) are configured for traversing a patient's body part and wherein the mounting sites are configured to be located on different sides of the traversed body part.

8. The medical positioning device (2) of claim 6 when dependent on claim 5, wherein each support member (14) is mounted to the respective carriage (8) via the respective spherical joint (16).

9. The medical positioning device (2) of any one of claims 6-8, wherein the at least one support member (14) is C-shaped, U-shaped, V-shaped or T-shaped.

10. The medical positioning device (2) of any one of claims 6-9, wherein the at least one support member (14) comprises at least one bracket (36) configured for mounting the respective pin (4) at the respective mounting site.

11. The medical positioning device (2) of claim 10, wherein the bracket's (36) position on the support member (14) is adjustable.

12. The medical positioning device (2) of claim 10 or 11, wherein the brackets (36) are configured to induce a pretension into the pin (4) using a three point fixation at each mounting site.

13. The medical positioning device (2) of any one of the preceding claims, further configured for repositioning bones in a patient's body part, preferably for repositioning bones in a human's foot.

14. The medical positioning device (2) of any one of claims 5-13, wherein the area (A) generated by the base frame (10) is free of the pins (4).

15. The medical positioning device (2) of any one of claims 6-14, wherein the extension assembly (24) is associated with the first carriage assembly (6a) and forms a first extension assembly (24), comprising:
a threaded rod (26) coupled to the support member (14a) of the first carriage assembly (6a), wherein the medical positioning device (2) is configured for adjusting the distance (D2) between the first and second pins (4a, 4b) by rotational movement between the threaded rod (26) and a counter threading.

16. The medical positioning device (2) of any one of the preceding claims, wherein the second carriage assembly (6a) comprises a force sensor (42).

17. The medical positioning device (2) of any one of claims 1-16, wherein the first carriage assembly (6a) comprises the extension assembly (24).

18. The medical positioning device (2) of any one of claims 6-17 when dependent on claim 5, wherein each of the spherical joints (16) is arranged to couple the respective support member (14) to the respective carriage (8), and wherein preferably at least one spherical joint (16), more preferably each of the spherical joints (16), comprises a ball (18), a corresponding socket (20) and a locking mechanism configured for reversibly receiving and locking the ball (18) in the socket (20).

## Patentansprüche

1. Medizinische Positioniervorrichtung (2), die aufweist:
eine erste Trägeranordnung (6a), die zur Befestigung eines ersten Stifts (4a) konfiguriert ist, wobei die Ausrichtung des ersten Stifts (4a) mittels eines ersten Kugelgelenks (16a) mit drei Rotationsfreiheitsgraden mit einem Winkelbereich von jeweils mindestens ±10° einstellbar ist;
eine zweite Trägeranordnung (6b), die zur Befestigung eines zweiten Stifts (4b) konfiguriert ist, wobei die Ausrichtung des zweiten Stifts (4b) mittels eines zweiten Kugelgelenks (16b) mit drei Rotationsfreiheitsgraden mit einem Winkelbereich von jeweils mindestens ±10° einstellbar ist;
eine Streckanordnung (24), die zum Einstellen eines Abstands (D2) zwischen dem ersten Stift (4a) und dem zweiten Stift (4b) konfiguriert ist;
wobei nur eine der Trägeranordnungen (6) eine Streckanordnung (24) aufweist.

2. Medizinische Positioniervorrichtung (2) nach Anspruch 1, wobei die Streckanordnung (24) zum Einstellen eines Abstands (D2) zwischen der ersten Trägeranordnung (6a) und der zweiten Trägeranordnung (6b) konfiguriert ist, wodurch der Abstand zwischen dem ersten Stift (4a) und dem zweiten Stift (4b) eingestellt wird.

3. Medizinische Positioniervorrichtung (2) nach Anspruch 1 oder 2, wobei die Streckanordnung (24) zum Einstellen des Abstands zwischen dem ersten und dem zweiten Stift relativ zueinander in einem Bereich von mindestens 2 cm oder mindestens 4 cm und/oder bis zu 10 cm oder bis zu 8 cm oder bis zu 5 cm konfiguriert ist.

4. Medizinische Positioniervorrichtung (2) nach einem der vorhergehenden Ansprüche, wobei die erste Trägeranordnung (6a) und/oder die zweite Trägeranordnung (6b) konfiguriert sind, einem Moment von mindestens 3 Nm, vorzugsweise mindestens 9,5 Nm, besonders bevorzugt mindestens 11 Nm, ganz besonders bevorzugt mindestens 12,5 Nm zu widerstehen, das von den Stiften (4a, 4b) ausgeübt wird.

5. Medizinische Positioniervorrichtung (2) nach einem der vorhergehenden Ansprüche, die ferner einen Grundrahmen (10) aufweist, wobei die erste Trägeranordnung (6a) und die zweite Trägeranordnung (6b) über einen ersten bzw. einen zweiten Träger (8) verstellbar am Grundrahmen (10) befestigt sind; oder wobei eine der ersten und zweiten Trägeranordnung (6) über den jeweiligen Träger (8) verstellbar an dem Grundrahmen (10) befestigt ist und die andere Trägeranordnung (6) über den jeweiligen Träger (8) nicht verstellbar an dem Grundrahmen (10) befestigt ist

6. Medizinische Positioniervorrichtung (2) nach einem der vorhergehenden Ansprüche, wobei mindestens eine der ersten und zweiten Trägeranordnung (6) ein Halteelement (14) aufweist, das zur Befestigung des jeweiligen Stifts (4) an zwei Befestigungsstellen konfiguriert ist, wobei die Befestigungsstellen vorzugsweise 7 bis 20 cm, besonders bevorzugt 10 bis 14 cm voneinander entfernt sind.

7. Medizinische Positionierungsvorrichtung (2) nach Anspruch 6, wobei die Stifte (4) zum Durchqueren eines Körperteils eines Patienten konfiguriert sind und wobei die Befestigungsstellen konfiguriert sind, sich auf verschiedenen Seiten des durchquerten Körperteils zu befinden.

8. Medizinische Positioniervorrichtung (2) nach Anspruch 6, wenn von Anspruch 5 abhängig, wobei jedes Halteelement (14) über das jeweilige Kugelgelenk (16) an dem jeweiligen Träger (8) befestigt ist.

9. Medizinische Positioniervorrichtung (2) nach einem der Ansprüche 6 bis 8, wobei das mindestens eine Halteelement (14) C-förmig, U-förmig, V-förmig oder T-förmig ist.

10. Medizinische Positioniervorrichtung (2) nach einem der Ansprüche 6 bis 9, wobei das mindestens eine Halteelement (14) mindestens eine Halterung (36) aufweist, die zur Befestigung des jeweiligen Stifts (4) an der jeweiligen Befestigungsstelle konfiguriert ist.

11. Medizinische Positionierungsvorrichtung (2) nach Anspruch 10, wobei die Position der Halterung (36) auf dem Halteelement (14) einstellbar ist.

12. Medizinische Positioniervorrichtung (2) nach Anspruch 10 oder 11, wobei die Halterungen (36) konfiguriert sind, unter Verwendung einer Dreipunktbefestigung an jeder Befestigungsstelle eine Vorspannung in den Stift (4) zu induzieren.

13. Medizinische Positionierungsvorrichtung (2) nach einem der vorhergehenden Ansprüche, die ferner zur Neupositionierung von Knochen in einem Körperteil eines Patienten, vorzugsweise zur Neupositionierung von Knochen in einem menschlichen Fuß, konfiguriert ist.

14. Medizinische Positionierungsvorrichtung (2) nach einem der Ansprüche 5 bis 13, wobei die durch den Grundrahmen (10) gebildete Bereich (A) frei von den Stiften (4) ist.

15. Medizinische Positioniervorrichtung (2) nach einem der Ansprüche 6 bis 14, wobei die Streckanordnung (24) mit der ersten Trägeranordnung (6a) assoziiert ist und eine erste Streckanordnung (24) bildet, die aufweist:
eine Gewindestange (26), die mit dem Halteelement (14a) der ersten Trägeranordnung (6a) gekoppelt ist, wobei die medizinische Positioniervorrichtung (2) zum Einstellen des Abstands (D2) zwischen dem ersten und dem zweiten Stift (4a, 4b) durch eine Drehbewegung zwischen der Gewindestange (26) und einem Gegengewinde konfiguriert ist.

16. Medizinische Positioniervorrichtung (2) nach einem der vorhergehenden Ansprüche, wobei die zweite Trägeranordnung (6a) einen Kraftsensor (42) aufweist.

17. Medizinische Positioniervorrichtung (2) nach einem der Ansprüche 1 bis 16, wobei die erste Trägeranordnung (6a) die Streckanordnung (24) aufweist.

18. Medizinische Positioniervorrichtung (2) nach einem der Ansprüche 6 bis 17, wenn von Anspruch 5 abhängig, wobei jedes der Kugelgelenke (16) angeordnet ist, das jeweilige Halteelement (14) mit dem jeweiligen Träger (8) zu koppeln, und wobei vorzugsweise mindestens ein Kugelgelenk (16), besonders bevorzugt jedes der Kugelgelenke (16), eine Kugel (18), eine entsprechende Pfanne (20) und einen Verriegelungsmechanismus aufweist, der konfiguriert ist, die Kugel (18) in der Pfanne (20) reversibel aufzunehmen und zu verriegeln.

## Revendications

1. Dispositif de positionnement médical (2), comprenant :
un premier ensemble de chariot (6a) prévu pour monter une première broche (4a), l'orientation de la première broche (4a) étant réglable au moyen d'une première articulation sphérique (16a) à trois degrés de liberté de rotation avec une plage angulaire d'au moins ±10° chacun ;
un deuxième ensemble de chariot (6b) prévu pour monter une deuxième broche (4b), l'orientation de la deuxième broche (4b) étant réglable au moyen d'une deuxième articulation sphérique (16b) avec trois degrés de liberté de rotation avec une plage angulaire d'au moins ±10° chacun ;
un ensemble d'extension (24) prévu pour régler une distance (D2) entre la première broche (4a) et la deuxième broche (4b) ;
où un seul des ensembles de chariots (6) comprend un ensemble d'extension (24).

2. Dispositif de positionnement médical (2) selon la revendication 1, où l'ensemble d'extension (24) est prévu pour régler une distance (D2) entre le premier ensemble de chariot (6a) et le deuxième ensemble de chariot (6b), en réglant ainsi la distance entre la première broche (4a) et la deuxième broche (4b).

3. Dispositif de positionnement médical (2) selon la revendication 1 ou la revendication 2, où l'ensemble d'extension (24) est prévu pour régler la distance entre la première et la deuxième broches l'une par rapport à l'autre dans une plage d'au moins 2 cm, ou d'au moins 4 cm, et/ou jusqu'à 10 cm, ou jusqu'à 8 cm, ou jusqu'à 5 cm.

4. Dispositif de positionnement médical (2) selon l'une des revendications précédentes, où le premier ensemble de chariot (6a) et/ou le deuxième ensemble de chariot (6b) sont prévus pour résister à un couple d'au moins 3 Nm, avantageusement d'au moins 9,5 Nm, de préférence d'au moins 11 Nm, tout particulièrement d'au moins 12,5 Nm, appliqué respectivement par les broches (4a, 4b).

5. Dispositif de positionnement médical (2) selon l'une des revendications précédentes, comprenant en outre un cadre de base (10), où le premier ensemble de chariot (6a) et le deuxième ensemble de chariot (6b) sont montés de manière réglable sur le cadre de base (10) respectivement au moyen du premier et du deuxième chariots (8) ; ou où un ensemble entre le premier et le deuxième ensembles de chariot (6) est monté de manière réglable sur le cadre de base (10) au moyen du chariot correspondant (8) et l'autre ensemble de chariot (6) est monté de manière non réglable sur le cadre de base (10) au moyen du chariot correspondant (8).

6. Dispositif de positionnement médical (2) selon l'une des revendications précédentes, où le premier et/ou le deuxième ensembles de chariot (6) comprennent un élément de support (14) prévu pour monter la broche correspondante (4) sur deux sites de montage, les sites de montage étant avantageusement séparés de 7 à 20 cm, de préférence de 10 à 14 cm.

7. Dispositif de positionnement médical (2) selon la revendication 6, où les broches (4) sont prévues pour traverser une partie du corps d'un patient et où les sites de montage sont prévus pour être localisés sur des côtés différents de la partie du corps traversée.

8. Dispositif de positionnement médical (2) selon la revendication 6, si dépendante de la revendication 5, où chaque élément de support (14) est monté sur le chariot correspondant (8) au moyen de l'articulation sphérique correspondante (16).

9. Dispositif de positionnement médical (2) selon l'une des revendications 6 à 8, où ledit au moins un élément de support (14) est en forme de C, de U, de V ou de T.

10. Dispositif de positionnement médical (2) selon l'une des revendications 6 à 9, où ledit au moins un élément de support (14) comprend au moins un support (36) prévu pour monter la broche correspondante (4) sur le site de montage correspondant.

11. Dispositif de positionnement médical (2) selon la revendication 10, où la position du support (36) sur l'élément de support (14) est réglable.

12. Dispositif de positionnement médical (2) selon la revendication 10 ou la revendication 11, où les supports (36) sont prévus pour induire une précontrainte sur la broche (4) au moyen d'une fixation en trois points sur chaque site de montage.

13. Dispositif de positionnement médical (2) selon l'une des revendications précédentes, prévu en outre pour repositionner les os d'une partie du corps d'un patient, de préférence pour repositionner les os d'un pied humain.

14. Dispositif de positionnement médical (2) selon l'une des revendications 5 à 13, où la zone (A) générée par le cadre de base (10) est exempte de broches (4).

15. Dispositif de positionnement médical (2) selon l'une des revendications 6 à 14, où l'ensemble d'extension (24) est associé au premier ensemble de chariot (6a) et forme un premier ensemble d'extension (24), comprenant :
une tige filetée (26) accouplée à l'élément de support (14a) du premier ensemble de chariot (6a), le dispositif de positionnement médical (2) étant prévu pour régler la distance (D2) entre la première et la deuxième broches (4a, 4b) par un mouvement de rotation entre la tige filetée (26) et un contre-filet.

16. Dispositif de positionnement médical (2) selon l'une des revendications précédentes, où le deuxième ensemble de chariot (6a) comprend un capteur de force (42).

17. Dispositif de positionnement médical (2) selon l'une des revendications 1 à 16, où le premier ensemble de chariot (6a) comprend l'ensemble d'extension (24).

18. Dispositif de positionnement médical (2) selon l'une des revendications 6 à 17, si dépendante de la revendication 5, où chacune des articulations sphériques (16) est agencée pour accoupler l'élément de support correspondant (14) au chariot correspondant (8), et où avantageusement au moins une articulation sphérique (16), de préférence chacune des articulations sphériques (16), comprend une bille (18), une douille correspondante (20) et un mécanisme de verrouillage prévu pour recevoir et verrouiller de manière réversible la bille (18) dans la douille (20).
